# EUROPEAN PATENT APPLICATION

(11) **EP 2 599 878 A1**
(43) Date of publication of application: **05.06.2013**
(21) Application number: 11812385.0
(22) Date of filing: 22.07.2011
(51) Int. Cl.: C12Q 1/68, C12N 15/09, G01N 33/58

(54) **DETECTION METHOD OF NOVEL RET FUSION**

(30) Priority: 26.07.2010 JP 2010166674
(71) Applicant: Astellas Pharma Inc., Tokyo 103-8411 (JP)
(72) Inventor: SHINDO, Nobuaki, Tokyo 103-8411 (JP); NISHIMURA, Kouichi, Tokyo 103-8411 (JP); SANO, Yorikata, Tokyo 103-8411 (JP); ASAUMI, Makoto, Tokyo 103-8411 (JP); YAMANAKA, Aya, Tokyo 103-8411 (JP); FUTAMI, Takashi, Tokyo 103-8411 (JP); KAWASE, Tatsuya, Tokyo 103-8411 (JP)
(74) Representative: HOFFMANN EITLE
(86) International application number: PCT/JP2011/066661
(87) International publication number: WO 2012/014795

(57) **Abstract**

[Problem]

An object of the present invention is to define a polynucleotide as a novel gene responsible for cancer, thereby providing detection method of the polynucleotide or a polypeptide encoded by the polynucleotide and kits as well as primer sets for the method.

[Means for Solution]

It was found that a fusion gene of a portion of KIF5B gene and a portion of RET gene, which is present in a portion of cancer patients, is a gene responsible for cancer. Based on the finding, detection methods of a polynucleotide as the gene and a polypeptide encoded by the polynucleotide were established. In the detection methods, a fusion gene of a portion of KIF5B gene and a portion of RET gene, or a fusion protein encoded by the fusion gene are detected. The primer sets and the detection kits comprise sense primers designed from a portion encoding KIF5B and antisense primers designed from a portion encoding RET.

SEQUENCE LISTING

<110>  Astellas Pharma Inc.

<120>  Method for detection of a novel ret fusions

<130>  A11022A00

<150>  JP 2010-166674
<151>  2010-07-26

<160>  12

<170>  PatentIn version 3.5

<210>  1
<211>  2808
<212>  DNA
<213>  Homo sapiens



<220>
<221>  CDS
<222>  (1)..(2808)

<400>  1
atg gcg gac ctg gcc gag tgc aac atc aaa gtg atg tgt cgc ttc aga      48
Met Ala Asp Leu Ala Glu Cys Asn Ile Lys Val Met Cys Arg Phe Arg
1               5                   10                  15

cct ctc aac gag tct gaa gtg aac cgc ggc gac aag tac atc gcc aag      96
Pro Leu Asn Glu Ser Glu Val Asn Arg Gly Asp Lys Tyr Ile Ala Lys
            20                  25                  30

ttt cag gga gaa gac acg gtc gtg atc gcg tcc aag cct tat gca ttt      144
Phe Gln Gly Glu Asp Thr Val Val Ile Ala Ser Lys Pro Tyr Ala Phe
        35                  40                  45

gat cgg gtg ttc cag tca agc aca tct caa gag caa gtg tat aat gac      192
Asp Arg Val Phe Gln Ser Ser Thr Ser Gln Glu Gln Val Tyr Asn Asp
    50                  55                  60

tgt gca aag aag att gtt aaa gat gta ctt gaa gga tat aat gga aca      240
Cys Ala Lys Lys Ile Val Lys Asp Val Leu Glu Gly Tyr Asn Gly Thr
65                  70                  75                  80

ata ttt gca tat gga caa aca tcc tct ggg aag aca cac aca atg gag      288
Ile Phe Ala Tyr Gly Gln Thr Ser Ser Gly Lys Thr His Thr Met Glu
                85                  90                  95

ggt aaa ctt cat gat cca gaa ggc atg gga att att cca aga ata gtg      336
Gly Lys Leu His Asp Pro Glu Gly Met Gly Ile Ile Pro Arg Ile Val
            100                 105                 110

caa gat att ttt aat tat att tac tcc atg gat gaa aat ttg gaa ttt      384
Gln Asp Ile Phe Asn Tyr Ile Tyr Ser Met Asp Glu Asn Leu Glu Phe
        115                 120                 125

cat att aag gtt tca tat ttt gaa ata tat ttg gat aag ata agg gac      432
His Ile Lys Val Ser Tyr Phe Glu Ile Tyr Leu Asp Lys Ile Arg Asp
    130                 135                 140

ctg tta gat gtt tca aag acc aac ctt tca gtt cat gaa gac aaa aac      480
Leu Leu Asp Val Ser Lys Thr Asn Leu Ser Val His Glu Asp Lys Asn
145                 150                 155                 160

cga gtt ccc tat gta aag ggg tgc aca gag cgt ttt gta tgt agt cca 528
Arg Val Pro Tyr Val Lys Gly Cys Thr Glu Arg Phe Val Cys Ser Pro
                165                 170                 175

gat gaa gtt atg gat acc ata gat gaa gga aaa tcc aac aga cat gta 576
Asp Glu Val Met Asp Thr Ile Asp Glu Gly Lys Ser Asn Arg His Val
            180                 185                 190

gca gtt aca aat atg aat gaa cat agc tct agg agt cac agt ata ttt 624
Ala Val Thr Asn Met Asn Glu His Ser Ser Arg Ser His Ser Ile Phe
        195                 200                 205

ctt att aat gtc aaa caa gag aac aca caa acg gaa caa aag ctg agt 672
Leu Ile Asn Val Lys Gln Glu Asn Thr Gln Thr Glu Gln Lys Leu Ser
    210                 215                 220

gga aaa ctt tat ctg gtt gat tta gct ggt agt gaa aag gtt agt aaa 720
Gly Lys Leu Tyr Leu Val Asp Leu Ala Gly Ser Glu Lys Val Ser Lys
225                 230                 235                 240

act gga gct gaa ggt gct gtg ctg gat gaa gct aaa aac atc aac aag 768
Thr Gly Ala Glu Gly Ala Val Leu Asp Glu Ala Lys Asn Ile Asn Lys
                245                 250                 255

tca ctt tct gct ctt gga aat gtt att tct gct ttg gct gag ggt agt 816
Ser Leu Ser Ala Leu Gly Asn Val Ile Ser Ala Leu Ala Glu Gly Ser
            260                 265                 270

aca tat gtt cca tat cga gat agt aaa atg aca aga atc ctt caa gat 864
Thr Tyr Val Pro Tyr Arg Asp Ser Lys Met Thr Arg Ile Leu Gln Asp
        275                 280                 285

tca tta ggt ggc aac tgt aga acc act att gta att tgt tgc tct cca 912
Ser Leu Gly Gly Asn Cys Arg Thr Thr Ile Val Ile Cys Cys Ser Pro
    290                 295                 300

tca tca tac aat gag tct gaa aca aaa tct aca ctc tta ttt ggc caa 960
Ser Ser Tyr Asn Glu Ser Glu Thr Lys Ser Thr Leu Leu Phe Gly Gln
305                 310                 315                 320

agg gcc aaa aca att aag aac aca gtt tgt gtc aat gtg gag tta act 1008
Arg Ala Lys Thr Ile Lys Asn Thr Val Cys Val Asn Val Glu Leu Thr
                325                 330                 335

gca gaa cag tgg aaa aag aag tat gaa aaa gaa aaa gaa aaa aat aag 1056
Ala Glu Gln Trp Lys Lys Lys Tyr Glu Lys Glu Lys Glu Lys Asn Lys
            340                 345                 350

atc ctg cgg aac act att cag tgg ctt gaa aat gag ctc aac aga tgg 1104
Ile Leu Arg Asn Thr Ile Gln Trp Leu Glu Asn Glu Leu Asn Arg Trp
        355                 360                 365

cgt aat ggg gag acg gtg cct att gat gaa cag ttt gac aaa gag aaa 1152
Arg Asn Gly Glu Thr Val Pro Ile Asp Glu Gln Phe Asp Lys Glu Lys
    370                 375                 380

gcc aac ttg gaa gct ttc aca gtg gat aaa gat att act ctt acc aat 1200
Ala Asn Leu Glu Ala Phe Thr Val Asp Lys Asp Ile Thr Leu Thr Asn
385                 390                 395                 400

gat aaa cca gca acc gca att gga gtt ata gga aat ttt act gat gct 1248
Asp Lys Pro Ala Thr Ala Ile Gly Val Ile Gly Asn Phe Thr Asp Ala
                405                 410                 415

gaa aga aga aag tgt gaa gaa gaa att gct aaa tta tac aaa cag ctt 1296
Glu Arg Arg Lys Cys Glu Glu Glu Ile Ala Lys Leu Tyr Lys Gln Leu
            420                 425                 430

gat gac aag gat gaa gaa att aac cag caa agt caa ctg gta gag aaa 1344
Asp Asp Lys Asp Glu Glu Ile Asn Gln Gln Ser Gln Leu Val Glu Lys
        435                 440                 445

ctg aag acg caa atg ttg gat cag gag gag ctt ttg gca tct acc aga 1392
Leu Lys Thr Gln Met Leu Asp Gln Glu Glu Leu Leu Ala Ser Thr Arg
    450                 455                 460

agg gat caa gac aat atg caa gct gag ctg aat cgc ctt caa gca gaa 1440
Arg Asp Gln Asp Asn Met Gln Ala Glu Leu Asn Arg Leu Gln Ala Glu
465                 470                 475                 480

aat gat gcc tct aaa gaa gaa gtg aaa gaa gtt tta cag gcc cta gaa 1488
Asn Asp Ala Ser Lys Glu Glu Val Lys Glu Val Leu Gln Ala Leu Glu
                485                 490                 495

gaa ctt gct gtc aat tat gat cag aag tct cag gaa gtt gaa gac aaa 1536
Glu Leu Ala Val Asn Tyr Asp Gln Lys Ser Gln Glu Val Glu Asp Lys
            500                 505                 510

act aag gaa tat gaa ttg ctt agt gat gaa ttg aat cag aaa tcg gca 1584
Thr Lys Glu Tyr Glu Leu Leu Ser Asp Glu Leu Asn Gln Lys Ser Ala
        515                 520                 525

act tta gcg agt ata gat gct gag ctt cag aaa ctt aag gaa atg acc 1632
Thr Leu Ala Ser Ile Asp Ala Glu Leu Gln Lys Leu Lys Glu Met Thr
    530                 535                 540

aac cac cag aaa aaa cga gca gct gag atg atg gca tct tta cta aaa 1680
Asn His Gln Lys Lys Arg Ala Ala Glu Met Met Ala Ser Leu Leu Lys
545                 550                 555                 560

gac ctt gca gaa ata gga att gct gtg gga aat aat gat gta aag gag 1728
Asp Leu Ala Glu Ile Gly Ile Ala Val Gly Asn Asn Asp Val Lys Glu
                565                 570                 575

gat cca aag tgg gaa ttc cct cgg aag aac ttg gtt ctt gga aaa act 1776
Asp Pro Lys Trp Glu Phe Pro Arg Lys Asn Leu Val Leu Gly Lys Thr
            580                 585                 590

cta gga gaa ggc gaa ttt gga aaa gtg gtc aag gca acg gcc ttc cat 1824
Leu Gly Glu Gly Glu Phe Gly Lys Val Val Lys Ala Thr Ala Phe His
        595                 600                 605

ctg aaa ggc aga gca ggg tac acc acg gtg gcc gtg aag atg ctg aaa 1872
Leu Lys Gly Arg Ala Gly Tyr Thr Thr Val Ala Val Lys Met Leu Lys
    610                 615                 620

gag aac gcc tcc ccg agt gag ctt cga gac ctg ctg tca gag ttc aac 1920
Glu Asn Ala Ser Pro Ser Glu Leu Arg Asp Leu Leu Ser Glu Phe Asn
625                 630                 635                 640

gtc ctg aag cag gtc aac cac cca cat gtc atc aaa ttg tat ggg gcc 1968
Val Leu Lys Gln Val Asn His Pro His Val Ile Lys Leu Tyr Gly Ala
                645                 650                 655

tgc agc cag gat ggc ccg ctc ctc ctc atc gtg gag tac gcc aaa tac 2016
Cys Ser Gln Asp Gly Pro Leu Leu Leu Ile Val Glu Tyr Ala Lys Tyr
            660                 665                 670

ggc tcc ctg cgg ggc ttc ctc cgc gag agc cgc aaa gtg ggg cct ggc 2064

Gly Ser Leu Arg Gly Phe Leu Arg Glu Ser Arg Lys Val Gly Pro Gly
        675                 680                 685

tac ctg ggc agt gga ggc agc cgc aac tcc agc tcc ctg gac cac ccg        2112
Tyr Leu Gly Ser Gly Gly Ser Arg Asn Ser Ser Ser Leu Asp His Pro
    690                 695                 700

gat gag cgg gcc ctc acc atg ggc gac ctc atc tca ttt gcc tgg cag        2160
Asp Glu Arg Ala Leu Thr Met Gly Asp Leu Ile Ser Phe Ala Trp Gln
705                 710                 715                 720

atc tca cag ggg atg cag tat ctg gcc gag atg aag ctc gtt cat cgg        2208
Ile Ser Gln Gly Met Gln Tyr Leu Ala Glu Met Lys Leu Val His Arg
                725                 730                 735

gac ttg gca gcc aga aac atc ctg gta gct gag ggg cgg aag atg aag        2256
Asp Leu Ala Ala Arg Asn Ile Leu Val Ala Glu Gly Arg Lys Met Lys
            740                 745                 750

att tcg gat ttc ggc ttg tcc cga gat gtt tat gaa gag gat tcc tac        2304
Ile Ser Asp Phe Gly Leu Ser Arg Asp Val Tyr Glu Glu Asp Ser Tyr
        755                 760                 765

gtg aag agg agc cag ggt cgg att cca gtt aaa tgg atg gca att gaa        2352
Val Lys Arg Ser Gln Gly Arg Ile Pro Val Lys Trp Met Ala Ile Glu
    770                 775                 780

tcc ctt ttt gat cat atc tac acc acg caa agt gat gta tgg tct ttt        2400
Ser Leu Phe Asp His Ile Tyr Thr Thr Gln Ser Asp Val Trp Ser Phe
785                 790                 795                 800

ggt gtc ctg ctg tgg gag atc gtg acc cta ggg gga aac ccc tat cct        2448
Gly Val Leu Leu Trp Glu Ile Val Thr Leu Gly Gly Asn Pro Tyr Pro
                805                 810                 815

ggg att cct cct gag cgg ctc ttc aac ctt ctg aag acc ggc cac cgg        2496
Gly Ile Pro Pro Glu Arg Leu Phe Asn Leu Leu Lys Thr Gly His Arg
            820                 825                 830

atg gag agg cca gac aac tgc agc gag gag atg tac cgc ctg atg ctg        2544
Met Glu Arg Pro Asp Asn Cys Ser Glu Glu Met Tyr Arg Leu Met Leu
        835                 840                 845

caa tgc tgg aag cag gag ccg gac aaa agg ccg gtg ttt gcg gac atc        2592
Gln Cys Trp Lys Gln Glu Pro Asp Lys Arg Pro Val Phe Ala Asp Ile
    850                 855                 860

agc aaa gac ctg gag aag atg atg gtt aag agg aga gac tac ttg gac        2640
Ser Lys Asp Leu Glu Lys Met Met Val Lys Arg Arg Asp Tyr Leu Asp
865                 870                 875                 880

ctt gcg gcg tcc act cca tct gac tcc ctg att tat gac gac ggc ctc        2688
Leu Ala Ala Ser Thr Pro Ser Asp Ser Leu Ile Tyr Asp Asp Gly Leu
                885                 890                 895

tca gag gag gag aca ccg ctg gtg gac tgt aat aat gcc ccc ctc cct        2736
Ser Glu Glu Glu Thr Pro Leu Val Asp Cys Asn Asn Ala Pro Leu Pro
            900                 905                 910

cga gcc ctc cct tcc aca tgg att gaa aac aaa ctc tat ggt aga att        2784
Arg Ala Leu Pro Ser Thr Trp Ile Glu Asn Lys Leu Tyr Gly Arg Ile
        915                 920                 925

tcc cat gca ttt act aga ttc tag                                        2808
Ser His Ala Phe Thr Arg Phe

930                 935

<210>  2
<211>  935
<212>  PRT
<213>  Homo sapiens

<400>  2

Met Ala Asp Leu Ala Glu Cys Asn Ile Lys Val Met Cys Arg Phe Arg
1               5                   10                  15

Pro Leu Asn Glu Ser Glu Val Asn Arg Gly Asp Lys Tyr Ile Ala Lys
            20                  25                  30

Phe Gln Gly Glu Asp Thr Val Val Ile Ala Ser Lys Pro Tyr Ala Phe
        35                  40                  45

Asp Arg Val Phe Gln Ser Ser Thr Ser Gln Glu Gln Val Tyr Asn Asp
    50                  55                  60

Cys Ala Lys Lys Ile Val Lys Asp Val Leu Glu Gly Tyr Asn Gly Thr
65                  70                  75                  80

Ile Phe Ala Tyr Gly Gln Thr Ser Ser Gly Lys Thr His Thr Met Glu
                85                  90                  95

Gly Lys Leu His Asp Pro Glu Gly Met Gly Ile Ile Pro Arg Ile Val
            100                 105                 110

Gln Asp Ile Phe Asn Tyr Ile Tyr Ser Met Asp Glu Asn Leu Glu Phe
        115                 120                 125

His Ile Lys Val Ser Tyr Phe Glu Ile Tyr Leu Asp Lys Ile Arg Asp
    130                 135                 140

Leu Leu Asp Val Ser Lys Thr Asn Leu Ser Val His Glu Asp Lys Asn
145                 150                 155                 160

Arg Val Pro Tyr Val Lys Gly Cys Thr Glu Arg Phe Val Cys Ser Pro
                165                 170                 175

Asp Glu Val Met Asp Thr Ile Asp Glu Gly Lys Ser Asn Arg His Val
            180                 185                 190

Ala Val Thr Asn Met Asn Glu His Ser Ser Arg Ser His Ser Ile Phe
        195                 200                 205

Leu Ile Asn Val Lys Gln Glu Asn Thr Gln Thr Glu Gln Lys Leu Ser
    210                 215                 220

Gly Lys Leu Tyr Leu Val Asp Leu Ala Gly Ser Glu Lys Val Ser Lys
225                 230                 235                 240

Thr Gly Ala Glu Gly Ala Val Leu Asp Glu Ala Lys Asn Ile Asn Lys
                245                 250                 255

Ser Leu Ser Ala Leu Gly Asn Val Ile Ser Ala Leu Ala Glu Gly Ser
            260                 265                 270

Thr Tyr Val Pro Tyr Arg Asp Ser Lys Met Thr Arg Ile Leu Gln Asp
        275                 280                 285

Ser Leu Gly Gly Asn Cys Arg Thr Thr Ile Val Ile Cys Cys Ser Pro
    290                 295                 300

Ser Ser Tyr Asn Glu Ser Glu Thr Lys Ser Thr Leu Leu Phe Gly Gln
305                 310                 315                 320

Arg Ala Lys Thr Ile Lys Asn Thr Val Cys Val Asn Val Glu Leu Thr
                325                 330                 335

Ala Glu Gln Trp Lys Lys Lys Tyr Glu Lys Glu Lys Glu Lys Asn Lys
            340                 345                 350

Ile Leu Arg Asn Thr Ile Gln Trp Leu Glu Asn Glu Leu Asn Arg Trp
        355                 360                 365

Arg Asn Gly Glu Thr Val Pro Ile Asp Glu Gln Phe Asp Lys Glu Lys
    370                 375                 380

Ala Asn Leu Glu Ala Phe Thr Val Asp Lys Asp Ile Thr Leu Thr Asn
385                 390                 395                 400

Asp Lys Pro Ala Thr Ala Ile Gly Val Ile Gly Asn Phe Thr Asp Ala
                405                 410                 415

Glu Arg Arg Lys Cys Glu Glu Glu Ile Ala Lys Leu Tyr Lys Gln Leu
            420                 425                 430

Asp Asp Lys Asp Glu Glu Ile Asn Gln Gln Ser Gln Leu Val Glu Lys
        435                 440                 445

Leu Lys Thr Gln Met Leu Asp Gln Glu Glu Leu Leu Ala Ser Thr Arg
    450                 455                 460

Arg Asp Gln Asp Asn Met Gln Ala Glu Leu Asn Arg Leu Gln Ala Glu
465                 470                 475                 480

Asn Asp Ala Ser Lys Glu Glu Val Lys Glu Val Leu Gln Ala Leu Glu
                485                 490                 495

Glu Leu Ala Val Asn Tyr Asp Gln Lys Ser Gln Glu Val Glu Asp Lys
            500                 505                 510

Thr Lys Glu Tyr Glu Leu Leu Ser Asp Glu Leu Asn Gln Lys Ser Ala
        515                 520                 525

Thr Leu Ala Ser Ile Asp Ala Glu Leu Gln Lys Leu Lys Glu Met Thr
    530                 535                 540

Asn His Gln Lys Lys Arg Ala Ala Glu Met Met Ala Ser Leu Leu Lys
545                 550                 555                 560

Asp Leu Ala Glu Ile Gly Ile Ala Val Gly Asn Asn Asp Val Lys Glu
                565                 570                 575

Asp Pro Lys Trp Glu Phe Pro Arg Lys Asn Leu Val Leu Gly Lys Thr
            580                 585                 590

Leu Gly Glu Gly Glu Phe Gly Lys Val Val Lys Ala Thr Ala Phe His
        595                 600                 605

Leu Lys Gly Arg Ala Gly Tyr Thr Thr Val Ala Val Lys Met Leu Lys
    610                 615                 620

Glu Asn Ala Ser Pro Ser Glu Leu Arg Asp Leu Leu Ser Glu Phe Asn
625                 630                 635                 640

Val Leu Lys Gln Val Asn His Pro His Val Ile Lys Leu Tyr Gly Ala
                645                 650                 655

Cys Ser Gln Asp Gly Pro Leu Leu Leu Ile Val Glu Tyr Ala Lys Tyr
            660                 665                 670

Gly Ser Leu Arg Gly Phe Leu Arg Glu Ser Arg Lys Val Gly Pro Gly
        675                 680                 685

Tyr Leu Gly Ser Gly Gly Ser Arg Asn Ser Ser Ser Leu Asp His Pro
    690                 695                 700

Asp Glu Arg Ala Leu Thr Met Gly Asp Leu Ile Ser Phe Ala Trp Gln
705                 710                 715                 720

Ile Ser Gln Gly Met Gln Tyr Leu Ala Glu Met Lys Leu Val His Arg
                725                 730                 735

Asp Leu Ala Ala Arg Asn Ile Leu Val Ala Glu Gly Arg Lys Met Lys

740                 745                 750

Ile Ser Asp Phe Gly Leu Ser Arg Asp Val Tyr Glu Glu Asp Ser Tyr
        755                 760                 765

Val Lys Arg Ser Gln Gly Arg Ile Pro Val Lys Trp Met Ala Ile Glu
    770                 775                 780

Ser Leu Phe Asp His Ile Tyr Thr Thr Gln Ser Asp Val Trp Ser Phe
785                 790                 795                 800

Gly Val Leu Leu Trp Glu Ile Val Thr Leu Gly Gly Asn Pro Tyr Pro
                805                 810                 815

Gly Ile Pro Pro Glu Arg Leu Phe Asn Leu Leu Lys Thr Gly His Arg
            820                 825                 830

Met Glu Arg Pro Asp Asn Cys Ser Glu Glu Met Tyr Arg Leu Met Leu
        835                 840                 845

Gln Cys Trp Lys Gln Glu Pro Asp Lys Arg Pro Val Phe Ala Asp Ile
    850                 855                 860

Ser Lys Asp Leu Glu Lys Met Met Val Lys Arg Arg Asp Tyr Leu Asp
865                 870                 875                 880

Leu Ala Ala Ser Thr Pro Ser Asp Ser Leu Ile Tyr Asp Asp Gly Leu
                885                 890                 895

Ser Glu Glu Glu Thr Pro Leu Val Asp Cys Asn Asn Ala Pro Leu Pro
            900                 905                 910

Arg Ala Leu Pro Ser Thr Trp Ile Glu Asn Lys Leu Tyr Gly Arg Ile
        915                 920                 925

Ser His Ala Phe Thr Arg Phe
    930                 935

<210>  3
<211>  2934
<212>  DNA
<213>  Homo sapiens

<220>
<221>  CDS
<222>  (1)..(2934)

<400>  3
atg gcg gac ctg gcc gag tgc aac atc aaa gtg atg tgt cgc ttc aga      48
Met Ala Asp Leu Ala Glu Cys Asn Ile Lys Val Met Cys Arg Phe Arg
1               5                   10                  15

cct ctc aac gag tct gaa gtg aac cgc ggc gac aag tac atc gcc aag     96
Pro Leu Asn Glu Ser Glu Val Asn Arg Gly Asp Lys Tyr Ile Ala Lys
        20                  25                  30

ttt cag gga gaa gac acg gtc gtg atc gcg tcc aag cct tat gca ttt     144
Phe Gln Gly Glu Asp Thr Val Val Ile Ala Ser Lys Pro Tyr Ala Phe
    35                  40                  45

gat cgg gtg ttc cag tca agc aca tct caa gag caa gtg tat aat gac     192
Asp Arg Val Phe Gln Ser Ser Thr Ser Gln Glu Gln Val Tyr Asn Asp
    50                  55                  60

tgt gca aag aag att gtt aaa gat gta ctt gaa gga tat aat gga aca     240
Cys Ala Lys Lys Ile Val Lys Asp Val Leu Glu Gly Tyr Asn Gly Thr
65                  70                  75                  80

ata ttt gca tat gga caa aca tcc tct ggg aag aca cac aca atg gag     288
Ile Phe Ala Tyr Gly Gln Thr Ser Ser Gly Lys Thr His Thr Met Glu
                85                  90                  95

ggt aaa ctt cat gat cca gaa ggc atg gga att att cca aga ata gtg     336
Gly Lys Leu His Asp Pro Glu Gly Met Gly Ile Ile Pro Arg Ile Val
            100                 105                 110

caa gat att ttt aat tat att tac tcc atg gat gaa aat ttg gaa ttt     384
Gln Asp Ile Phe Asn Tyr Ile Tyr Ser Met Asp Glu Asn Leu Glu Phe
        115                 120                 125

cat att aag gtt tca tat ttt gaa ata tat ttg gat aag ata agg gac     432
His Ile Lys Val Ser Tyr Phe Glu Ile Tyr Leu Asp Lys Ile Arg Asp
    130                 135                 140

ctg tta gat gtt tca aag acc aac ctt tca gtt cat gaa gac aaa aac     480
Leu Leu Asp Val Ser Lys Thr Asn Leu Ser Val His Glu Asp Lys Asn
145                 150                 155                 160

cga gtt ccc tat gta aag ggg tgc aca gag cgt ttt gta tgt agt cca     528
Arg Val Pro Tyr Val Lys Gly Cys Thr Glu Arg Phe Val Cys Ser Pro
                165                 170                 175

gat gaa gtt atg gat acc ata gat gaa gga aaa tcc aac aga cat gta     576
Asp Glu Val Met Asp Thr Ile Asp Glu Gly Lys Ser Asn Arg His Val
            180                 185                 190

gca gtt aca aat atg aat gaa cat agc tct agg agt cac agt ata ttt     624
Ala Val Thr Asn Met Asn Glu His Ser Ser Arg Ser His Ser Ile Phe
        195                 200                 205

ctt att aat gtc aaa caa gag aac aca caa acg gaa caa aag ctg agt     672
Leu Ile Asn Val Lys Gln Glu Asn Thr Gln Thr Glu Gln Lys Leu Ser
    210                 215                 220

gga aaa ctt tat ctg gtt gat tta gct ggt agt gaa aag gtt agt aaa     720
Gly Lys Leu Tyr Leu Val Asp Leu Ala Gly Ser Glu Lys Val Ser Lys
225                 230                 235                 240

act gga gct gaa ggt gct gtg ctg gat gaa gct aaa aac atc aac aag     768
Thr Gly Ala Glu Gly Ala Val Leu Asp Glu Ala Lys Asn Ile Asn Lys
                245                 250                 255

tca ctt tct gct ctt gga aat gtt att tct gct ttg gct gag ggt agt     816
Ser Leu Ser Ala Leu Gly Asn Val Ile Ser Ala Leu Ala Glu Gly Ser
            260                 265                 270

aca tat gtt cca tat cga gat agt aaa atg aca aga atc ctt caa gat 864
Thr Tyr Val Pro Tyr Arg Asp Ser Lys Met Thr Arg Ile Leu Gln Asp
        275                 280                 285

tca tta ggt ggc aac tgt aga acc act att gta att tgc tgc tct cca 912
Ser Leu Gly Gly Asn Cys Arg Thr Thr Ile Val Ile Cys Cys Ser Pro
    290                 295                 300

tca tca tac aat gag tct gaa aca aaa tct aca ctc tta ttt ggc caa 960
Ser Ser Tyr Asn Glu Ser Glu Thr Lys Ser Thr Leu Leu Phe Gly Gln
305                 310                 315                 320

agg gcc aaa aca att aag aac aca gtt tgt gtc aat gtg gag tta act 1008
Arg Ala Lys Thr Ile Lys Asn Thr Val Cys Val Asn Val Glu Leu Thr
                325                 330                 335

gca gaa cag tgg aaa aag aag tat gaa aaa gaa aaa gaa aaa aat aag 1056
Ala Glu Gln Trp Lys Lys Lys Tyr Glu Lys Glu Lys Glu Lys Asn Lys
            340                 345                 350

atc ctg cgg aac act att cag tgg ctt gaa aat gag ctc aac aga tgg 1104
Ile Leu Arg Asn Thr Ile Gln Trp Leu Glu Asn Glu Leu Asn Arg Trp
        355                 360                 365

cgt aat ggg gag acg gtg cct att gat gaa cag ttt gac aaa gag aaa 1152
Arg Asn Gly Glu Thr Val Pro Ile Asp Glu Gln Phe Asp Lys Glu Lys
    370                 375                 380

gcc aac ttg gaa gct ttc aca gtg gat aaa gat att act ctt acc aat 1200
Ala Asn Leu Glu Ala Phe Thr Val Asp Lys Asp Ile Thr Leu Thr Asn
385                 390                 395                 400

gat aaa cca gca acc gca att gga gtt ata gga aat ttt act gat gct 1248
Asp Lys Pro Ala Thr Ala Ile Gly Val Ile Gly Asn Phe Thr Asp Ala
                405                 410                 415

gaa aga aga aag tgt gaa gaa gaa att gct aaa tta tac aaa cag ctt 1296
Glu Arg Arg Lys Cys Glu Glu Glu Ile Ala Lys Leu Tyr Lys Gln Leu
            420                 425                 430

gat gac aag gat gaa gaa att aac cag caa agt caa ctg gta gag aaa 1344
Asp Asp Lys Asp Glu Glu Ile Asn Gln Gln Ser Gln Leu Val Glu Lys
        435                 440                 445

ctg aag acg caa atg ttg gat cag gag gag ctt ttg gca tct acc aga 1392
Leu Lys Thr Gln Met Leu Asp Gln Glu Glu Leu Leu Ala Ser Thr Arg
    450                 455                 460

agg gat caa gac aat atg caa gct gag ctg aat cgc ctt caa gca gaa 1440
Arg Asp Gln Asp Asn Met Gln Ala Glu Leu Asn Arg Leu Gln Ala Glu
465                 470                 475                 480

aat gat gcc tct aaa gaa gaa gtg aaa gaa gtt tta cag gcc cta gaa 1488
Asn Asp Ala Ser Lys Glu Glu Val Lys Glu Val Leu Gln Ala Leu Glu
                485                 490                 495

gaa ctt gct gtc aat tat gat cag aag tct cag gaa gtt gaa gac aaa 1536
Glu Leu Ala Val Asn Tyr Asp Gln Lys Ser Gln Glu Val Glu Asp Lys
            500                 505                 510

act aag gaa tat gaa ttg ctt agt gat gaa ttg aat cag aaa tcg gca 1584
Thr Lys Glu Tyr Glu Leu Leu Ser Asp Glu Leu Asn Gln Lys Ser Ala
        515                 520                 525

act tta gcg agt ata gat gct gag ctt cag aaa ctt aag gaa atg acc 1632

Thr Leu Ala Ser Ile Asp Ala Glu Leu Gln Lys Leu Lys Glu Met Thr
    530                 535                 540

aac cac cag aaa aaa cga gca gct gag atg atg gca tct tta cta aaa      1680
Asn His Gln Lys Lys Arg Ala Ala Glu Met Met Ala Ser Leu Leu Lys
545                 550                 555                 560

gac ctt gca gaa ata gga att gct gtg gga aat aat gat gta aag gag      1728
Asp Leu Ala Glu Ile Gly Ile Ala Val Gly Asn Asn Asp Val Lys Glu
                565                 570                 575

gat cca aag tgg gaa ttc cct cgg aag aac ttg gtt ctt gga aaa act      1776
Asp Pro Lys Trp Glu Phe Pro Arg Lys Asn Leu Val Leu Gly Lys Thr
            580                 585                 590

cta gga gaa ggc gaa ttt gga aaa gtg gtc aag gca acg gcc ttc cat      1824
Leu Gly Glu Gly Glu Phe Gly Lys Val Val Lys Ala Thr Ala Phe His
        595                 600                 605

ctg aaa ggc aga gca ggg tac acc acg gtg gcc gtg aag atg ctg aaa      1872
Leu Lys Gly Arg Ala Gly Tyr Thr Thr Val Ala Val Lys Met Leu Lys
    610                 615                 620

gag aac gcc tcc ccg agt gag ctt cga gac ctg ctg tca gag ttc aac      1920
Glu Asn Ala Ser Pro Ser Glu Leu Arg Asp Leu Leu Ser Glu Phe Asn
625                 630                 635                 640

gtc ctg aag cag gtc aac cac cca cat gtc atc aaa ttg tat ggg gcc      1968
Val Leu Lys Gln Val Asn His Pro His Val Ile Lys Leu Tyr Gly Ala
                645                 650                 655

tgc agc cag gat ggc ccg ctc ctc ctc atc gtg gag tac gcc aaa tac      2016
Cys Ser Gln Asp Gly Pro Leu Leu Leu Ile Val Glu Tyr Ala Lys Tyr
            660                 665                 670

ggc tcc ctg cgg ggc ttc ctc cgc gag agc cgc aaa gtg ggg cct ggc      2064
Gly Ser Leu Arg Gly Phe Leu Arg Glu Ser Arg Lys Val Gly Pro Gly
        675                 680                 685

tac ctg ggc agt gga ggc agc cgc aac tcc agc tcc ctg gac cac ccg      2112
Tyr Leu Gly Ser Gly Gly Ser Arg Asn Ser Ser Ser Leu Asp His Pro
    690                 695                 700

gat gag cgg gcc ctc acc atg ggc gac ctc atc tca ttt gcc tgg cag      2160
Asp Glu Arg Ala Leu Thr Met Gly Asp Leu Ile Ser Phe Ala Trp Gln
705                 710                 715                 720

atc tca cag ggg atg cag tat ctg gcc gag atg aag ctc gtt cat cgg      2208
Ile Ser Gln Gly Met Gln Tyr Leu Ala Glu Met Lys Leu Val His Arg
                725                 730                 735

gac ttg gca gcc aga aac atc ctg gta gct gag ggg cgg aag atg aag      2256
Asp Leu Ala Ala Arg Asn Ile Leu Val Ala Glu Gly Arg Lys Met Lys
            740                 745                 750

att tcg gat ttc ggc ttg tcc cga gat gtt tat gaa gag gat tcg tac      2304
Ile Ser Asp Phe Gly Leu Ser Arg Asp Val Tyr Glu Glu Asp Ser Tyr
        755                 760                 765

gtg aag agg agc cag ggt cgg att cca gtt aaa tgg atg gca att gaa      2352
Val Lys Arg Ser Gln Gly Arg Ile Pro Val Lys Trp Met Ala Ile Glu
    770                 775                 780

tcc ctt ttt gat cat atc tac acc acg caa agt gat gta tgg tct ttt      2400
Ser Leu Phe Asp His Ile Tyr Thr Thr Gln Ser Asp Val Trp Ser Phe

785                 790                 795                 800

ggt gtc ctg ctg tgg gag atc gtg acc cta ggg gga aac ccc tat cct       2448
Gly Val Leu Leu Trp Glu Ile Val Thr Leu Gly Gly Asn Pro Tyr Pro
                805                 810                 815

ggg att cct cct gag cgg ctc ttc aac ctt ctg aag acc ggc cac cgg       2496
Gly Ile Pro Pro Glu Arg Leu Phe Asn Leu Leu Lys Thr Gly His Arg
            820                 825                 830

atg gag agg cca gac aac tgc agc gag gag atg tac cgc ctg atg ctg       2544
Met Glu Arg Pro Asp Asn Cys Ser Glu Glu Met Tyr Arg Leu Met Leu
        835                 840                 845

caa tgc tgg aag cag gag ccg gac aaa agg ccg gtg ttt gcg gac atc       2592
Gln Cys Trp Lys Gln Glu Pro Asp Lys Arg Pro Val Phe Ala Asp Ile
    850                 855                 860

agc aaa gac ctg gag aag atg atg gtt aag agg aga gac tac ttg gac       2640
Ser Lys Asp Leu Glu Lys Met Met Val Lys Arg Arg Asp Tyr Leu Asp
865                 870                 875                 880

ctt gcg gcg tcc act cca tct gac tcc ctg att tat gac gac ggc ctc       2688
Leu Ala Ala Ser Thr Pro Ser Asp Ser Leu Ile Tyr Asp Asp Gly Leu
                885                 890                 895

tca gag gag gag aca ccg ctg gtg gac tgt aat aat gcc ccc ctc cct       2736
Ser Glu Glu Glu Thr Pro Leu Val Asp Cys Asn Asn Ala Pro Leu Pro
            900                 905                 910

cga gcc ctc cct tcc aca tgg att gaa aac aaa ctc tat ggc atg tca       2784
Arg Ala Leu Pro Ser Thr Trp Ile Glu Asn Lys Leu Tyr Gly Met Ser
        915                 920                 925

gac ccg aac tgg cct gga gag agt cct gta cca ctc acg aga gct gat       2832
Asp Pro Asn Trp Pro Gly Glu Ser Pro Val Pro Leu Thr Arg Ala Asp
    930                 935                 940

ggc act aac act ggg ttt cca aga tat cca aat gat agt gta tat gct       2880
Gly Thr Asn Thr Gly Phe Pro Arg Tyr Pro Asn Asp Ser Val Tyr Ala
945                 950                 955                 960

aac tgg atg ctt tca ccc tca gcg gca aaa tta atg gac acg ttt gat       2928
Asn Trp Met Leu Ser Pro Ser Ala Ala Lys Leu Met Asp Thr Phe Asp
                965                 970                 975

agt taa                                                               2934
Ser

<210>  4
<211>  977
<212>  PRT
<213>  Homo sapiens

<400>  4

Met Ala Asp Leu Ala Glu Cys Asn Ile Lys Val Met Cys Arg Phe Arg
1               5                   10                  15

Pro Leu Asn Glu Ser Glu Val Asn Arg Gly Asp Lys Tyr Ile Ala Lys
            20                  25                  30

Phe Gln Gly Glu Asp Thr Val Val Ile Ala Ser Lys Pro Tyr Ala Phe
        35                  40                  45

Asp Arg Val Phe Gln Ser Ser Thr Ser Gln Glu Gln Val Tyr Asn Asp
    50                  55                  60

Cys Ala Lys Lys Ile Val Lys Asp Val Leu Glu Gly Tyr Asn Gly Thr
65                  70                  75                  80

Ile Phe Ala Tyr Gly Gln Thr Ser Ser Gly Lys Thr His Thr Met Glu
                85                  90                  95

Gly Lys Leu His Asp Pro Glu Gly Met Gly Ile Ile Pro Arg Ile Val
            100                 105                 110

Gln Asp Ile Phe Asn Tyr Ile Tyr Ser Met Asp Glu Asn Leu Glu Phe
        115                 120                 125

His Ile Lys Val Ser Tyr Phe Glu Ile Tyr Leu Asp Lys Ile Arg Asp
    130                 135                 140

Leu Leu Asp Val Ser Lys Thr Asn Leu Ser Val His Glu Asp Lys Asn
145                 150                 155                 160

Arg Val Pro Tyr Val Lys Gly Cys Thr Glu Arg Phe Val Cys Ser Pro
                165                 170                 175

Asp Glu Val Met Asp Thr Ile Asp Glu Gly Lys Ser Asn Arg His Val
            180                 185                 190

Ala Val Thr Asn Met Asn Glu His Ser Ser Arg Ser His Ser Ile Phe
        195                 200                 205

Leu Ile Asn Val Lys Gln Glu Asn Thr Gln Thr Glu Gln Lys Leu Ser
    210                 215                 220

Gly Lys Leu Tyr Leu Val Asp Leu Ala Gly Ser Glu Lys Val Ser Lys
225                 230                 235                 240

Thr Gly Ala Glu Gly Ala Val Leu Asp Glu Ala Lys Asn Ile Asn Lys
                245                 250                 255

Ser Leu Ser Ala Leu Gly Asn Val Ile Ser Ala Leu Ala Glu Gly Ser
            260                 265                 270

Thr Tyr Val Pro Tyr Arg Asp Ser Lys Met Thr Arg Ile Leu Gln Asp
        275                 280                 285

Ser Leu Gly Gly Asn Cys Arg Thr Thr Ile Val Ile Cys Cys Ser Pro
    290                 295                 300

Ser Ser Tyr Asn Glu Ser Glu Thr Lys Ser Thr Leu Leu Phe Gly Gln
305                 310                 315                 320

Arg Ala Lys Thr Ile Lys Asn Thr Val Cys Val Asn Val Glu Leu Thr
                325                 330                 335

Ala Glu Gln Trp Lys Lys Lys Tyr Glu Lys Glu Lys Glu Lys Asn Lys
            340                 345                 350

Ile Leu Arg Asn Thr Ile Gln Trp Leu Glu Asn Glu Leu Asn Arg Trp
        355                 360                 365

Arg Asn Gly Glu Thr Val Pro Ile Asp Glu Gln Phe Asp Lys Glu Lys
    370                 375                 380

Ala Asn Leu Glu Ala Phe Thr Val Asp Lys Asp Ile Thr Leu Thr Asn
385                 390                 395                 400

Asp Lys Pro Ala Thr Ala Ile Gly Val Ile Gly Asn Phe Thr Asp Ala
                405                 410                 415

Glu Arg Arg Lys Cys Glu Glu Glu Ile Ala Lys Leu Tyr Lys Gln Leu
            420                 425                 430

Asp Asp Lys Asp Glu Glu Ile Asn Gln Gln Ser Gln Leu Val Glu Lys
        435                 440                 445

Leu Lys Thr Gln Met Leu Asp Gln Glu Glu Leu Leu Ala Ser Thr Arg
    450                 455                 460

Arg Asp Gln Asp Asn Met Gln Ala Glu Leu Asn Arg Leu Gln Ala Glu
465                 470                 475                 480

Asn Asp Ala Ser Lys Glu Glu Val Lys Glu Val Leu Gln Ala Leu Glu
                485                 490                 495

Glu Leu Ala Val Asn Tyr Asp Gln Lys Ser Gln Glu Val Glu Asp Lys
            500                 505                 510

Thr Lys Glu Tyr Glu Leu Leu Ser Asp Glu Leu Asn Gln Lys Ser Ala
        515                 520                 525

Thr Leu Ala Ser Ile Asp Ala Glu Leu Gln Lys Leu Lys Glu Met Thr
    530                 535                 540

Asn His Gln Lys Lys Arg Ala Ala Glu Met Met Ala Ser Leu Leu Lys

545                 550                 555                 560

Asp Leu Ala Glu Ile Gly Ile Ala Val Gly Asn Asn Asp Val Lys Glu
                565                 570                 575

Asp Pro Lys Trp Glu Phe Pro Arg Lys Asn Leu Val Leu Gly Lys Thr
            580                 585                 590

Leu Gly Glu Gly Glu Phe Gly Lys Val Val Lys Ala Thr Ala Phe His
        595                 600                 605

Leu Lys Gly Arg Ala Gly Tyr Thr Thr Val Ala Val Lys Met Leu Lys
    610                 615                 620

Glu Asn Ala Ser Pro Ser Glu Leu Arg Asp Leu Leu Ser Glu Phe Asn
625                 630                 635                 640

Val Leu Lys Gln Val Asn His Pro His Val Ile Lys Leu Tyr Gly Ala
                645                 650                 655

Cys Ser Gln Asp Gly Pro Leu Leu Leu Ile Val Glu Tyr Ala Lys Tyr
            660                 665                 670

Gly Ser Leu Arg Gly Phe Leu Arg Glu Ser Arg Lys Val Gly Pro Gly
        675                 680                 685

Tyr Leu Gly Ser Gly Gly Ser Arg Asn Ser Ser Ser Leu Asp His Pro
    690                 695                 700

Asp Glu Arg Ala Leu Thr Met Gly Asp Leu Ile Ser Phe Ala Trp Gln
705                 710                 715                 720

Ile Ser Gln Gly Met Gln Tyr Leu Ala Glu Met Lys Leu Val His Arg
                725                 730                 735

Asp Leu Ala Ala Arg Asn Ile Leu Val Ala Glu Gly Arg Lys Met Lys
            740                 745                 750

Ile Ser Asp Phe Gly Leu Ser Arg Asp Val Tyr Glu Glu Asp Ser Tyr
        755                 760                 765

Val Lys Arg Ser Gln Gly Arg Ile Pro Val Lys Trp Met Ala Ile Glu
    770                 775                 780

Ser Leu Phe Asp His Ile Tyr Thr Thr Gln Ser Asp Val Trp Ser Phe
785                 790                 795                 800

Gly Val Leu Leu Trp Glu Ile Val Thr Leu Gly Gly Asn Pro Tyr Pro
                805                 810                 815

Gly Ile Pro Pro Glu Arg Leu Phe Asn Leu Leu Lys Thr Gly His Arg
            820                 825                 830

Met Glu Arg Pro Asp Asn Cys Ser Glu Glu Met Tyr Arg Leu Met Leu
        835                 840                 845

Gln Cys Trp Lys Gln Glu Pro Asp Lys Arg Pro Val Phe Ala Asp Ile
    850                 855                 860

Ser Lys Asp Leu Glu Lys Met Met Val Lys Arg Arg Asp Tyr Leu Asp
865                 870                 875                 880

Leu Ala Ala Ser Thr Pro Ser Asp Ser Leu Ile Tyr Asp Asp Gly Leu
                885                 890                 895

Ser Glu Glu Glu Thr Pro Leu Val Asp Cys Asn Asn Ala Pro Leu Pro
            900                 905                 910

Arg Ala Leu Pro Ser Thr Trp Ile Glu Asn Lys Leu Tyr Gly Met Ser
        915                 920                 925

Asp Pro Asn Trp Pro Gly Glu Ser Pro Val Pro Leu Thr Arg Ala Asp
    930                 935                 940

Gly Thr Asn Thr Gly Phe Pro Arg Tyr Pro Asn Asp Ser Val Tyr Ala
945                 950                 955                 960

Asn Trp Met Leu Ser Pro Ser Ala Ala Lys Leu Met Asp Thr Phe Asp
                965                 970                 975

Ser

<210>  5
<211>  35
<212>  DNA
<213>  Artificial

<220>
<223>  Description of Artificial Sequence:an artificially systhesized
       primer sequence

<400>  5
gcggccgcaa gatggcggac ctggccgagt gcaac                                35

<210>  6
<211>  35
<212>  DNA
<213>  Artificial

<220>

<223>  Description of Artificial Sequence:an artificially systhesized
      primer sequence

<400>  6
gcggccgcct agaatctagt aaatgcatgg gaaat                                35


<210>  7
<211>  35
<212>  DNA
<213>  Artificial

<220>
<223>  Description of Artificial Sequence:an artificially systhesized
      primer sequence

<400>  7
gcggccgctt aactatcaaa cgtgtccatt aattt                                35


<210>  8
<211>  71
<212>  DNA
<213>  Artificial

<220>
<223>  Description of Artificial Sequence:an artificially systhesized
      primer sequence

<400>  8
gtggcggccg caccatggac tacaaagacg atgacgacaa gcttgcggac ctggccgagt      60

gcaacatcaa a                                                          71


<210>  9
<211>  25
<212>  DNA
<213>  Homo sapiens

<400>  9
aaagaccttg cagaaatagg aattg                                           25


<210>  10
<211>  22
<212>  DNA
<213>  Homo sapiens

<400>  10
gaattcccac tttggatcct cc                                              22


<210>  11
<211>  36
<212>  DNA
<213>  Artificial

<220>
<223>  Description of Artificial Sequence:an artificially systhesized
      primer sequence

<400>  11
caccactagt atggactaca aagacgatga cgacaa                               36

<210> 12
<211> 39
<212> DNA
<213> Artificial

<220>
<223> Description of Artificial Sequence:an artificially systhesized
      primer sequence

<400> 12
aatgtcgacc tagaatctag taaatgcatg ggaaattct 39

## Description

### [Technical Field]

The present invention relates to a detection method of a novel fusion gene that has an RET kinase domain or a fusion protein that is encoded by the fusion gene.

### [Background Art]

A rearranged during transformation (RET) gene is mapped near the centromere of the long arm of chromosome 10 and consists of 21 exons. A protein encoded by this gene is receptor-type tyrosine kinase. The RET gene has a transmembrane domain in the central portion, a tyrosine kinase domain on a carboxyl terminal and an extracellular domain on an amino terminal. It is known that there are three types of proteins including 1072 amino acids (RET 9), 1106 amino acids (RET 43) and 1114 amino acids (RET 51) due to the different splicing pattern in the carboxyl terminal (Non-Patent Document 1). By forming a dimer via a ligand/GFR complex, RET phosphorylates and activates its own tyrosine (Non-Patent Document 1).

It is reported that the RET gene is fused with other genes (H4, ELE1 and the like) due to intrachromosomal inversion or interchromosomal translocation, whereby fusion tyrosine kinase RET/PTC having an transforming activity is expressed in papillary thyroid cancer (Non-Patent Document 2). The fusion is caused on the amino terminal in the tyrosine kinase domain of RET, and RET loses the extracellular domain and the transmembrane domain and forms a fusion protein with a fusion partner. Since many of the fusion partners have a domain for forming a complex, the fusion protein itself is also considered to form a complex, and it is considered that the formation of the complex causes the constitutive autophosphorylation of tyrosine kinase activity of RET, and intracellular signaling is aberrantly activated, which induces oncogenic transformation and cell growth (Non-Patent Document 2). Actually, when the RET/PTC fusion gene is transduced into normal mouse NIH3T3 cells, the cells are transformed into cells like cancer cells, and an RET kinase inhibitor can inhibit the cell growth of a thyroid cancer cell line expressing the RET/PTC fusion gene. These facts suggest that the RET/PTC fused is likely to be a target molecule in treating thyroid cancer (Non-Patent Documents 3 to 5). So far, it has been reported that 10 or more kinds of RET/PTC fusion genes are found in thyroid cancer, but there is no report showing that the RET fusion genes are found in lung cancer.

A kinesin family protein 5B (KIF5B) gene is mapped in the short arm of chromosome 10 and consists of 26 exons. It is known that KIF5B is one of the molecules constituting a motor protein complex involved in microtubule transport in a cell, and it is involved in the intracellular transport of materials with a characteristic of moving along the microtubule (Non-Patent Document 6).

So far, it has been reported that a fusion protein of KIF5B and receptor-type tyrosine kinase PDGFRα is expressed in a patient with hypereosinophilia (Non-Patent Document 7). In addition, it is reported that a fusion protein of KIF5B and Anaplastic Lymphoma Kinase as receptor-type tyrosine kinase is found in non-small cell lung cancer (Non-Patent Document 8), but there is no report showing that KIF5B is fused with RET.

### [Related Art]

### [Non-Patent Document]

[Non-Patent Document 1] TRENDS in Genetics, (UK), 2006, Vol. 22, p. 627-636
[Non-Patent Document 2] European journal of endoclinology, (UK), 2006, Vol. 155, p. 645-653
[Non-Patent Document 3] CANCER RESEARCH, (USA), 2002, Vol. 62, p. 7284-7290
[Non-Patent Document 4] The journal of Clinical Endocrinology & Metabolism, (USA), 2006, Vol. 91, p. 4070-4076
[Non-Patent Document 5] THE JOURNAL OF BIOLOGICAL CHEMISTRY, (USA), 2007, Vol. 282, p. 29230-29340
[Non-Patent Document 6] NATURE REVIEWS Molecular cell Biology, (UK), 2009, Vol. 10, p. 877-884
[Non-Patent Document 7] Leukemia, (UK), 2006, Vol. 20, p. 827-832
[Non-Patent Document 8] CLINICAL CANCER RESEARCH, (USA), 2009, Vol. 15, p. 3143-3149

### [Disclosure of Invention]

### [Problem to Be Solved by the Invention]

An object of the present invention is to define a polynucleotide as a novel gene responsible for cancer, thereby providing a detection method of the polynucleotide or a polypeptide encoded by the polynucleotide and a kit as well as a primer set for the method.

### [Means for Solving the Problems]

The present inventors isolated and identified novel fusion genes in which a portion of KIF5B gene was fused with a portion of RET gene as kinase from specimens obtained from patients with lung cancer (Example 1). As a result, they found that those fusion genes are present in the specimen from the patient with lung cancer (Example 2), and the fusion genes have tumorigenicity and are genes responsible for cancer (Examples 3, 4 and 6). Based on the knowledge, the present inventors established detection methods of those fusion genes and fusion proteins encoded by the fusion genes (Examples 2 and 5), and provided kits and primer sets for the method. Detection of those fusion genes or the fusion proteins encoded by the fusion genes makes it possible to screen cancer patients to be subjects who will receive drug therapy using RET inhibitors.

That is, the present invention relates to the following [1] to [9].
[1] A detection method of a fusion gene of Kinesin family protein 5B (KIF5B) gene and rearranged during transformation (RET) gene or a fusion protein encoded by the fusion gene, comprising:
   a step of detecting the presence of a polynucleotide encoding the following polypeptide or presence of the polypeptide in a sample obtained from a test subject:
   a polypeptide that comprises an amino acid sequence having 90% or higher identities with the amino acid sequence represented by SEQ ID NO: 2 or 4 and has tumorigenicity.
[2] The method according to [1],
   wherein the polypeptide is a polypeptide that comprises the amino acid sequence represented by SEQ ID NO: 2 or 4 and has tumorigenicity, or a polypeptide that comprises an amino acid sequence formed by deletion, substitution and/or insertion of 1 to 10 amino acids in the amino acid sequence represented by SEQ ID NO: 2 or 4 and has tumorigenicity.
[3] The method according to [1] or [2],
   wherein the polypeptide is a polypeptide consisting of the amino acid sequence represented by SEQ ID NO: 2 or 4.
[4] A kit for detecting a fusion gene of KIF5B gene and RET gene, comprising:
   sense and antisense primers designed so as to be able to specifically amplify a polynucleotide encoding the following polypeptide:
      a polypeptide that comprises an amino acid sequence having 90% or higher identities with the amino acid sequence represented by SEQ ID NO: 2 or 4 and has tumorigenicity.
[5] The kit according to [4],
   wherein the polypeptide is a polypeptide that comprises the amino acid sequence represented by SEQ ID NO: 2 or 4 and has tumorigenicity, or a polypeptide that comprises an amino acid sequence formed by deletion, substitution and/or insertion of 1 to 10 amino acids in the amino acid sequence represented by SEQ ID NO: 2 or 4 and has tumorigenicity.
[6] The kit according to [4] or [5],
   wherein the polypeptide is a polypeptide consisting of the amino acid sequence represented by SEQ ID NO: 2 or 4.
[7] A primer set for detecting a fusion gene of KIF5B gene and RET gene, comprising:
   a sense primer designed from a portion encoding the KIF5B gene; and
   an antisense primer designed from a portion encoding the RET gene,
   wherein the antisense primer consists of a nucleic acid molecule that is hybridized with a polynucleotide encoding the polypeptide according to any one of [1] to [3] under a stringent condition, and
   the sense primer consists of a nucleic acid molecule that is hybridized with a complementary strand of the polynucleotide under a stringent condition.
[8] A primer set of
   a sense primer consisting of an oligonucleotide with at least any 16 consecutive bases located at base numbers 1 and 1725 of SEQ ID NO: 1 and
   an antisense primer consisting of an oligonucleotide complementary to an oligonucleotide with at least any 16 consecutive bases located at base numbers 1726 and 2808 of SEQ ID NO: 1, or a primer set consisting of complementary strands thereof,
   wherein a size of an amplification product amplified by the sense primer and the antisense primer is 1 kb or less.
[9] A primer set of
   a sense primer consisting of an oligonucleotide with at least any 16 consecutive bases located at base numbers 1 and 1725 of SEQ ID NO: 3 and
   an antisense primer consisting of an oligonucleotide complementary to an oligonucleotide with at least any 16 consecutive bases located at base numbers 1726 and 2934 of SEQ ID NO: 3, or a primer set consisting of complementary strands thereof,
   wherein a size of an amplification product amplified by the sense primer and the antisense primer is 1 kb or less.

The present invention also relates to a detection method of the presence of cancer (particularly lung cancer) positive for a fusion gene having RET kinase domain, including:
a step of detecting the presence of a polynucleotide that encodes polypeptides according to the following sections (1) to (3) in a sample obtained from a test subject,
   (1) a polypeptide that comprises the amino acid sequence represented by SEQ ID NO: 2 or 4 and has tumorigenicity, or a polypeptide that comprises an amino acid sequence formed by deletion, substitution and/or insertion of 1 to 10 amino acids in the amino acid sequence represented by SEQ ID NO: 2 or 4 and has tumorigenicity,
   (2) a polypeptide that comprises an amino acid sequence having 90% or higher identities with the amino acid sequence represented by SEQ ID NO: 2 or 4 and has tumorigenicity, or
   (3) a polypeptide that consists of the amino acid sequence represented by SEQ ID NO: 2 or 4.

### [Effects of the Invention]

The detection methods of the present invention can be used as a method of detecting cancer (particularly lung cancer) positive for a fusion gene of KIF5B gene and RET gene. The detection methods of the present invention can also be used as a method of detecting chromosomal rearrangement. In addition, according to the detection methods of the present invention, it is possible to determine whether or not a patient is a test subject who will be administered with RET inhibitors, and as a result, it is possible to provide tailor-made treatment in which RET inhibitor therapy is expected to be effective. The detection kits and primer sets of the present invention can be used for the detection method of the present invention.

### [Embodiments for Carrying Out the Invention]

### <Detection method of the present invention>

The detection method of the present invention includes (1) a detection method of a fusion gene, and (2) a detection method of a fusion protein encoded by the fusion gene.

### (1) Detection method of fusion gene

The detection method of a fusion gene of the present invention includes a step of detecting the presence of polynucleotides of the present specification in a sample obtained from a test subject.

As the sample obtained from a test subject, substances collected from a test subject (samples isolated from a living body), specifically, any types of body fluid (preferably blood) collected, alveolar and bronchial washings, samples having undergone biopsy, and phlegm samples are used. Preferably, a biopsy sample or a phlegm sample from an affected area in the lung of a test subject is used. From the sample, genome DNA can be extracted and used. In addition, transcripts thereof (products produced as a result of transcription and translation of a genome; for example, mRNA, cDNA and proteins) can be used. Particularly, it is preferable to prepare and use mRNA or cDNA.

In the "step of detecting the presence of polynucleotides of the present specification" in the detection method of a fusion gene, the polynucleotides of the present specification to be detected (hereinafter, called polynucleotides to be detected) refer to polynucleotides that encode polypeptides as fusion proteins of a portion of KIF5B and a portion of RET having RET kinase domain. As the polynucleotides to be detected, polynucleotides encoding polypeptides according to the following sections (i) to (iii) are particularly preferable,
(i) a polypeptide that comprises the amino acid sequence represented by SEQ ID NO: 2 or 4 and has tumorigenicity, or a polypeptide that comprises an amino acid sequence formed by deletion, substitution and/or insertion of 1 to 10 amino acids in the amino acid sequence represented by SEQ ID NO: 2 or 4 and has tumorigenicity (hereinafter, called a functionally equivalent variant),
(ii) a polypeptide that comprises an amino acid sequence having 90% or higher identities with the amino acid sequence represented by SEQ ID NO: 2 or 4 and has tumorigenicity (hereinafter, called a homologous polypeptide), and
(iii) a polypeptide that consists of the amino acid sequence represented by SEQ ID NO: 2 or 4.

The amino acid sequence represented by SEQ ID NO: 2 is a sequence encoded by the base sequence represented by SEQ ID NO: 1. The amino acid sequence represented by SEQ ID NO: 4 is a sequence encoded by the base sequence represented by SEQ ID NO: 3. A protein which is a polypeptide consisting of the amino acids represented by SEQ ID NO: 2 is called a "KIF5B-RET fusion polypeptide v1S", and a protein which is a polypeptide consisting of the amino acids represented by SEQ ID NO: 4 is called a "KIF5B-RET fusion polypeptide v1L". The "KIF5B-RET fusion polypeptide v1S" and the "KIF5B-RET fusion polypeptide v1L" are collectively called a "KIF5B-RET fusion polypeptide v1". A gene encoding the KIF5B-RET fusion polypeptide v1S is called a "KIF5B-RET fusion polynucleotide v1S", and a gene encoding the KIF5B-RET fusion polypeptide v1L is called a "KIF5B-RET fusion polynucleotide v1L". A gene encoding the KIF5B-RET fusion polypeptide v1 is called a "KIF5B-RET fusion polynucleotide v1".

As a "functionally equivalent variant", a "polypeptide that comprises an amino acid sequence formed by deletion, substitution and/or insertion of 1 to 10 amino acids, preferably 1 to several amino acids, more preferably 1 to 7 amino acids, and most preferably 1 to 5 amino acids in the amino acid sequence represented by SEQ ID NO: 2 or 4 and has tumorigenicity" is preferable, and a "polypeptide that comprises the amino acid sequence represented by SEQ ID NO: 2 or 4 and has tumorigenicity" is particularly preferable.

A "homologous polypeptide" is a "polypeptide that comprises an amino acid sequence having 90% or higher identities with the amino acid sequence represented by SEQ ID NO: 2 or 4 and has tumorigenicity". As the homologous polypeptide, a polypeptide that comprises an amino acid sequence preferably having 95% or higher identities and more preferably having 98% or higher identities is preferable.

In addition, the "identities" in the present specification refers to Identities which is a value obtained using parameters prepared as default parameters by the search using NEEDLE program (J Mol Biol 1970; 48: 443-453). The parameters are as follows.
Gap penalty = 10
Extend penalty = 0.5
Matrix = EBLOSUM62

Whether a certain polypeptide has "tumorigenicity" is confirmed by the method of Example 4. Specifically, a host (3T3 fibroblast cell) into which a plasmid expressing the polypeptide has been transduced is subcutaneously inoculated into nude mice, and whether a tumor is formed is determined to confirm tumorigenicity.

As the polynucleotide to be detected in the detection method of the present invention, a polynucleotide encoding "a polypeptide that comprises the amino acid sequence represented by SEQ ID NO: 2 or 4 and has tumorigenicity" is preferable, and a polynucleotide encoding "a polypeptide that consists of the amino acid sequence represented by SEQ ID NO: 2 or 4" is most preferable.

The "step of detecting the presence of a polynucleotide" in the detection method of a polynucleotide of the present invention is performed by detecting the presence of a polynucleotide to be detected (genome sequence including a fusion point) in a genome of a sample obtained from a test subject, or by preparing a transcript (for example, mRNA or cDNA) of genome DNA extracted from a sample obtained from a test subject and detecting the presence of mRNA or cDNA corresponding to the polynucleotide to be detected.

Genome DNA can be extracted by known methods, and the extraction can be easily performed using a commercially available DNA extraction kit.

The step of detection can be performed according to known gene analysis methods (for example, known methods that are commonly used as gene detection methods such as PCR, LCR (Ligase chain reaction), SDA (Strand displacement amplification), NASBA (Nucleic acid sequence-based amplification), ICAN (Isothermal and chimeric primer-initiated amplification of nucleic acids), LAMP (Loop-mediated isothermal amplification) method, TMA (Gen-Probe's TMA system) method, in situ hybridization method, and microarrays). For example, a hybridization technology in which a nucleic acid hybridized with a polynucleotide to be detected is used as a probe, a gene amplification technology in which DNA hybridized with a polynucleotide to be detected is used as a primer, or the like is used.

Specifically, the detection is performed using nucleic acids derived from a sample obtained from a test subject, for example, mRNA and the like. The amount of mRNA is measured by a method of gene amplification reaction by using primers that are designed so as to be able to specifically amplify the sequence of a polynucleotide to be detected. The primers used in the detection method of the present invention, or the primers included in the detection kit are not particularly limited as long as the primers can specifically amplify the sequence of a polynucleotide to be detected, and designed based on the base sequence of a polynucleotide to be detected. Primers used in the PCR amplification monitor method can be designed using primer design software (for example, Primer Express manufactured by PE Biosystems) and the like. In addition, since the larger the size of a PCR product is, the more the amplification efficiency worsens, it is appropriate for a sense primer and an antisense primer to be designed such that the size of amplification products obtained when mRNA or cDNA is amplified becomes 1 kb or less.

More specifically, a sense primer (5'-primer) is designed from a portion encoding KIF5B, and an antisense primer (3'-primer) is designed from a portion encoding RET. It is preferable to use the primer included in the detection kit of the present invention, and it is more preferable to use the primer that is most suitably included in the detection kit. In the PCR amplification monitor method, it is also possible to design multiplex PCR for detecting all fusion polynucleotides in a single reaction liquid, by mixing the above sense primers corresponding to respective genes. By the method suitable for each amplification technology, it is possible to confirm whether or not a target gene (whole gene or a specific portion thereof) has been amplified. For example, in the PCR method, PCR products are analyzed by agarose gel electrophoresis and subjected to ethidium bromide staining and the like, whereby it is possible to confirm whether or not amplified fragments having a target size have been obtained. When the amplified fragments having a target size have been obtained, this indicates that a polynucleotide to be detected is present in the sample obtained from a test subject. The presence of a polynucleotide to be detected can be detected in this manner.

The detection method of a fusion gene of the present invention preferably includes a step of detecting the presence of a specific polynucleotide in a sample obtained from a test subject by a gene amplification reaction and a step of detecting whether or not amplified fragments having a target size have been obtained.

The detection using a hybridization technology is performed using, for example, northern hybridization, dot blotting method, DNA microarray method, and RNA protection method. As probes used for hybridization, it is possible to use a probe which comprises sequences consisting of 16 bases respectively upstream and downstream of the fusion point as a center of a nucleic acid molecule consisting of at least 32 consecutive bases hybridizing with a polynucleotide to be detected or with a complementary strand thereof in a stringent condition (preferably in a more stringent condition), or comprises complementary strands thereof.

The detection using an in situ hybridization technology can be performed according to a known FISH method (fusion assay). In addition, the detection can be performed by a fusion assay which is a combination of chromogenic in situ hybridization (CISH) method and silver in situ hybridization (SISH) method.

Moreover, the "fusion point" in the present specification refers to a point where a portion derived from the respective genes of KIF5B is fused with a portion derived from the RET gene.

Further, the "stringent condition" in the present specification refer to "5×SSPE, 5×Denhardt's solution, 0.5% SDS, 50% formamide, 200 µg/mL salmon sperm DNA, 42°C overnight" as conditions for hybridization, and "0.5xSSC, 0.1% SDS, 42°C" as conditions for washing. The "more stringent condition" refer to "5×SSPE, 5×Denhardt's solution, 0.5% SDS, 50% formamide, 200 µg/mL salmon sperm DNA, 42°C overnight" as conditions for hybridization, and "0.2xSSC, 0.1% SDS, 65°C" as conditions for washing.

It is also possible to use a gene amplification technology such as RT-PCR. In the RT-PCR method, the PCR amplification monitor (real time PCR) method (Genome Res., 6(10), 986, 1996) is performed during the process of gene amplification, whereby the presence of a polynucleotide to be detected can be more quantitatively analyzed. In the PCR amplification monitor method, for example, ABI PRISM7900 (manufactured by PE Biosystems) can be used. Real time PCR is a known method, and can be simply performed using commercially available instruments and kits for this method.

### (2) Detection method of fusion protein encoded by the above fusion gene

The detection method of a fusion protein of the present invention includes a step of detecting the presence of a specific polypeptide in a sample obtained from a test subject, that is, a polypeptide encoded by a polynucleotide to be detected (hereinafter, called a polypeptide to be detected).

Such a detection step can be performed by immunoassay method or enzyme activity assay method that is conducted by preparing a solubilized liquid derived from a sample obtained from a test subject (for example, a cancer tissue or cells obtained from a test subject) and combining a polypeptide to be detected contained in the liquid with an anti-KIF5B antibody and an anti-RET antibody. Preferably, it is possible to use techniques using a monoclonal antibody or a polyclonal antibody specific to a polypeptide to be detected, such as enzymatic immunoassay method, double antibodies sandwich ELISA method, fluorescence immunoassay method, radioimmunoassay method, and western blotting method.

When the polynucleotide to be detected or polypeptide to be detected in the detection method of the present invention is detected from a sample obtained from a test subject, the test subject is a subject (patient) who has cancer with the polynucleotide positive and is to be provided with treatment using RET inhibitors.

### <Detection kit of the present invention and primer set of the present invention>

The detection kit of the present invention comprises at least sense and antisense primers that are designed so as to be able to specifically amplify a polynucleotide to be detected in the detection method of the present invention. The sense and antisense primer set is a set of polynucleotides functioning as primers for amplifying a polynucleotide to be detected.

The "fusion gene of KIF5B gene and RET gene" refers to a gene in which a portion of the KIF5B gene is fused with a portion of the RET gene.

The primer set of the present invention comprises (1) a primer set which comprises a sense primer designed from a portion encoding KIF5B and an antisense primer designed from a portion encoding RET and is for detecting a fusion gene of KIF5B gene and RET gene, wherein the antisense primer consists of a nucleic acid molecule (preferably a nucleic acid molecule consisting of at least 16 bases) hybridizingwith a "polynucleotide to be detected" under a stringent condition (preferably under a more stringent condition), and the sense primer consists of a nucleic acid molecule (preferably a nucleic acid molecule consisting of at least 16 bases) hybridizing with a complementary strand of the "polynucleotide to be detected" under a stringent condition (preferably under a more stringent condition).

In addition, as a more specific embodiment of the primer set (1), the following primer sets (2) and (3) are included in the primer set of the present invention.
(2) A primer set of a sense primer that consists of an oligonucleotide consisting of at least any 16 consecutive bases between base numbers 1 and 1725 of SEQ ID NO: 1 and an antisense primer that consists of an oligonucleotide complementary to an oligonucleotide consisting of at least any 16 consecutive bases between base numbers 1726 and 2808 of SEQ ID NO: 1
(3) A primer set of a sense primer that consists of an oligonucleotide consisting of at least any 16 consecutive bases between base numbers 1 and 1725 of SEQ ID NO: 3 and an antisense primer that consists of an oligonucleotide complementary to an oligonucleotide consisting of at least any 16 consecutive bases between base numbers 1726 and 2934 of SEQ ID NO: 3

In these primer sets (1) to (3), an interval between the positions where the sense primer and the antisense primer are selected is preferably 1 kb or less, or the size of an amplification product amplified by the sense primer and the antisense primer is preferably 1 kb or less.

In addition, the primer of the present invention has a strand length consisting of 15 to 40 bases in general, preferably consisting of 16 to 24 bases, more preferably consisting of 18 to 24 bases, and particularly preferably consisting of 20 to 24 bases.

In the detection method of the present invention, the primer set of the present invention can be used for amplifying and detecting a polynucleotide to be detected. Moreover, though not particularly limited, the respective primers included in the primer set of the present invention can be prepared by, for example, chemical synthesis.

### [Examples]

Hereinafter, the present invention will be described in detail based on examples, but the present invention is not limited to the examples. In addition, unless otherwise specified, the present invention can be embodied according to known methods. Moreover, when commercially available reagents or kits are used, the present invention can be embodied according to the instruction of the commercially available products.

### [Example 1] Isolation of KIF5B-RET fusion polynucleotide v1

A reverse transcription reaction was performed on Lg 165 specimen RNA as RNA derived from a lung cancer tissue of a patient with non-small cell lung cancer (Asterand USA) by using reverse transcriptase (SuperScript III, Life Technologies) and oligo(dT) primer (Oligo(dT) 20 primer, Life Technologies), according to the protocol of the kit, thereby synthesizing cDNA.

Thereafter, by using primers of SEQ ID NOS: 5 and 6 including a recognition sequence for a restriction enzyme NotI, the cDNA obtained as above as a template, and a DNA polymerase (PrimeSTAR HS DNA Polymerase; TAKARA BIO INC.), PCR (98°C for 10 seconds, and then 60°C for 15 seconds and 68°C for 3.5 minutes, 35 cycles) was performed, followed by electrophoresis. As a result, it was found that about 3 kb of PCR products were obtained. The PCR products were digested with NotI and cloned into an NotI site present in a multicloning site of an expression vector (pTracer-CMV-bsd; Life Technologies), thereby establishing an expression plasmid. For insertion, the sequence was determined by dideoxy sequencing (BigDye Terminator v3.1 Cycle Sequencing Kit; Life Technologies). As a result, the presence of a transcript (KIF5B-RETv1S) (SEQ ID NO: 1) in which a portion from the N-terminal to exon 15 of a coding sequence (hereinafter, referred to as CDS) of KIF5B (NM_004521) registered in NCBI was fused with a portion from exon 12 of CDS to the C-terminal of CDS of a short form (NM_020630) of RET was clarified. A polypeptide encoded by SEQ ID NO: 1 is shown in SEQ ID NO: 2.

In addition, PCR was performed in the same manner as above by using primers of SEQ ID NOS: 5 and 7 including the NotI sequence, followed by cloning. As a result, the presence of a transcript (KIF5B-RETv1L) (SEQ ID NO: 3) in which a portion from the N-terminal to exon 15 of CDS of KIF5B registered in NCBI was fused with a portion from exon 12 of CDS to the C-terminal of CDS of a long form (NM_020975) of RET was observed. A polypeptide encoded by SEQ ID NO: 3 is shown in SEQ ID NO: 4.

Moreover, in order to express a full length ORF of KIF5B-RETv1S and KIF5B-RETv1L as a protein in which a FLAG tag is added to the N-terminal, by using primers of SEQ ID NOS: 8 and 6 into which the NotI site and the FLAG sequence were introduced and primers of SEQ ID NOS: 8 and 7, and using PCR products including the full length ORF of KIF5B-RETv1S and KIF5B-RETv1L as templates respectively, PCR was performed in the same conditions as above. The products were cloned into the NotI site present in the multicloning site of an expression vector (pTracer-CMV-bsd; Life Technologies), thereby establishing expression plasmids (FLAG-KIF5B-RETv1S/pTracer-CMV-bsd and FLAG-KIF5B-RETv1L/pTracer-CMV-bsd).

### [Example 2] Detection of KIF5B-RET fusion polynucleotide v1

By using cDNA 181 samples derived from clinical specimens of non-small cell lung cancer (Asterand USA) as substrates, oligonucleotides consisting of the base sequences represented by SEQ ID NOS: 9 and 10 as a primer set, a quantitative PCR kit (Power SYBR Green PCR Master Mix; Life Technologies), and Applied Biosystems 7900HT system, quantitative PCR (95°C for 10 minutes, and then 95°C for 15 seconds and 59°C for 60 seconds, 45 cycles) was performed to measure the gene expression level. As a result, amplification was confirmed only in the above sample Lg 165. The results show that by the above method, it is possible to detect the presence of the KIF5B-RET polynucleotide v1 in a sample derived from a clinical specimen of non-small cell lung cancer, and to screen a KIF5B-RET polynucleotide v1 positive patient.

### [Example 3] Examination of growth factor-independent growth acceleration effect of KIF5B-RET fusion polynucleotide v1

2 µg of the above FLAG-KIF5B-RETv1S/pTracer-CMV-bsd or FLAG-KIF5B-RETv1L/pTracer-CMV-bsd was introduced respectively into BA/F3 cells by using transfection reagent (Cell Line Nucleofector Kit V; Lonza) by electroporation (Nucleofector; Lonza), and cultured in RPMI1640 medium (Invitrogen) obtained by adding 10% bovine serum (Invitrogen) and 1 ng/mL of mouse recombinant IL-3 (R&D systems). Two days after the introduction, the medium was replaced with medium obtained by adding 10 µg/mL of Blastcidin (Invitrogen) to the above medium, and then the cells were cultured continuously for two more weeks. As a result, BA/F3 cells stably expressing KIF5B-RETv1S in which FLAG tag was added to the N-terminal and BA/F3 cells stably expressing KIF5B-RETv1L in which FLAG tag was added to the N-terminal (named FLAG-KIF5B-RETv1S expression/BA/F3 cells and FLAG-KIF5B-RETv1L expression/BA/F3 cells respectively) were obtained.

The growth potential of the FLAG-KIF5B-RETv1S expression/BA/F3 cells and FLAG-KIF5B-RETv1L expression/BA/F3 cells was examined. The FLAG-KIF5B-RETv1S expression/BA/F3 cells, FLAG-KIF5B-RETv1L expression/BA/F3 cells, and BA/F3 cells as the parent strain were seeded in a 96-well plate at 2×10³ cells per well and cultured for 8 days in the absence of growth factor IL-3, and cell counting reagent (CELLTITER-Gio™ Luminescent Cell Viability Assay; Promega Corporation) was added thereto, followed by stirring for 20 minutes. Thereafter, the cell number was measured using a luminescence measurement instrument (Envision; PerkinElmer Inc.). As a result, it was observed that though the BA/F3 cells died, the number of FLAG-KIF5B-RETv1S expression/BA/F3 cells and FLAG-KIF5B-RETv1L expression/BA/F3 cells was increased by 27 times and 26 times respectively compared to the day of seeding. From the above result, it was understood that the KIF5B-RET fusion polynucleotide v1S and KIF5B-RET fusion polynucleotide v1L have a growth factor-independent growth accelerating action.

### [Example 4] Examination of anchorage-independent growth accelerating action and tumorigenicity of KIF5B-RET fusion polynucleotide v1

In order to subclone the full length ORF of KIF5B-RETv1S into a lentiviral expression vector, the primer of SEQ ID NO: 11 into which an SpeI site was introduced and the primer of SEQ ID NO: 12 into which an SalI site was introduced were designed.

By using the above FLAG-KIF5B-RETv1S/pTracer-CMV-bsd as a template, PCR was performed (98°C for 10 seconds, and then 60°C for 15 seconds and 68°C for 3.5 minutes, 35 cycles) using PrimeSTAR HS DNA Polymerase (TAKARA BIO INC.), thereby obtaining PCR products of about 3 kb. The PCR products were purified (QIAquick Gel Extraction Kit; QIAGEN), and products obtained when the PCR products were digested with SpeI and SaiI were cloned into SpeI-XhoI site present in multicloning site of the lentiviral expression vector (pLenti 6.3/V5-TOPO; Life Technologies) (the resultant was named FLAG-KIF5B-RETv1 S/pLenti 6.3).

3 µg of the FLAG-KIF5B-RETv1S/pLenti 6.3 and 9 µg of the packaging plasmid (ViraPower™ Packaging Mix; Life Technologies) were introduced into 293FT cell packaging cells (Life Technologies) by using the transfection reagent (LipofectAmine 2000; Life Technologies). After 3 days, the culture supernatant was collected as lentivirus, Polybrene (Sigma-Aldrich Co. LLC.) was added thereto at a concentration of 6 µg/ml, and then mouse NIH3T3 cells were added thereto. After 2 days, the culture supernatant of the NIH3T3 cells was replaced to medium obtained by adding 10% bovine serum (Invitrogen) and 5 µg/ml of Blastcidin (Invivogen) to DMEM medium (Invitrogen), and the cells were continuously cultured for 2 more weeks. As a result, NIH3T3 cells (named FLAG-KIF5B-RETv1S/NIH3T3) stably expressing KIF5B-RETv1S in which FLAG was added to the N-terminal were obtained.

The FLAG-KIF5B-RETv1S/NIH3T3 cells and the NIH3T3 cells as the parent strain were seeded respectively into a medium containing 10% fetal bovine serum at 3×10³ cells per well in a 96-well spheroid plate (Sumiron Celltight Spheriod; SUMITOMO BAKELITE CO., LTD.). After the cells were cultured at 37°C in the presence of 5% CO₂, the number of cells obtained the next day (Day 1) and after 6 days (Day 6) was measured according to the method described in the manual of cell counting reagent (CellTiter-Glo™ Luminescent Cell Viability Assay; Promega Corporation). For detection, a luminescence measurement instrument (Envision; PerkinElmer Inc.) was used. It was confirmed that while the cell count of the NIH3T3 cells did not increase from Day 1 to Day 6, the cell count of the FLAG-KIF5B-RETv1S/NIH3T3 cells increased from Day 1 to Day 6. The result clearly showed that the growth of FLAG-KIF5B-RETv1S/NIH3T3 cells is anchorage-independent cell growth.

Subsequently, in order to examine in-vivo tumorigenicity, the present cell lines were transplanted into nude mice to examine tumorigenicity. The FLAG-KIF5B-RETv1S/NIH3T3 cells were subcutaneously inoculated into nude mice at 3×10⁶ cells, and the mice were observed for 8 days. As a result, the tumor formation was confirmed. This result showed that the KIF5B-RET fusion polynucleotide v1S is a gene responsible for cancer.

### [Example 5] Detection of KIF5B-RET fusion polypeptide v1S

A method of detecting the KIF5B-RET fusion polypeptide v1S in cells was established as follows. The FLAG-KIF5B-RETv1S/NIH3T3 cells were lysed in the cell lysis solution (50 mM Hepes pH 7.0, 150 mM NaCl, 1 mM EDTA, 2.5 mM EGTA, 10% glycerol, 0.1% Tween20, protease inhibitor (complete protease inhibitor cocktail; Roche Inc.)) and treated with sonication, followed by centrifugation. Thereafter, protein G beads (Protein G Sepharose 4 Fast Flow; GE Healthcare Inc.) and an anti-RET antibody (Cell Signaling Technology Inc.) or rabbit IgG (Cell Signaling Technology Inc.) were added to the obtained supernatant protein, followed by immunoprecipitation overnight at 4°C. After centrifugation, the precipitate was washed five times with the cell lysis solution and suspended in SDS lysis solution. Immunoblotting was performed on the supernatant thereof by using anti-KIF5B antibody (Sigma-Aldrich Co. LLC.). As a result, though detected in the product of immunoprecipitation performed using the anti-RET antibody, the KIF5B-RET fusion polypeptide v1S was not detected in the product of immunoprecipitation performed using the rabbit IgG. This result clearly showed that combining the anti-RET antibody with the anti-KIF5B antibody makes it possible to detect the presence of the KIF5B-RET fusion polypeptide v1S in cancer cells or cancer tissues expressing the KIF5B-RET fusion polypeptide v1S, and to determine cancer patients positive for KIF5B-RET fusion polypeptide v1S.

### [Example 6] Anchorage-independent cell growth inhibitory action of KIF5B-RET fusion polypeptide inhibitor against KIF5B-RET fusion polypeptide v1S-expressing 3T3 cell

The measurement (colony method or the like) of anchorage-independent cell growth is known to be a system for examining the anticancer action (pharmacological effect) of a compound ("Clinical Oncology" 2nd edition, Japanese Journal of Cancer & Chemotherapy). As a method that measures the growth of non-adherent cells unlike the colony method, there is a method using a spheroid plate as described above.

The FLAG-KIF5B-RETv1S/NIH3T3 cells were seeded at 2×10³ cells per well in a 96-well spheroid plate (Sumiron Celltight Spheriod; SUMITOMO BAKELITE CO., LTD.) by using a DMEM medium containing 10% fetal bovine serum. In addition, a plate to which only a medium was added was prepared as a positive control. After the cells were cultured overnight at 37°C in the presence of 5% CO₂, anti-cancer drug Sunitinib, Sorafenib or Vandetanib was added thereto at a final concentration of 1.1 µM. As a negative control, DMSO as solvent of the compound was added thereto at the same concentration (0.1 %) as the compound. Thereafter, the cells were cultured for 5 days at 37°C in the presence of 5% CO₂, and the cell counting reagent (CellTiter-Gio™ Luminescent Cell Viability Assay; Promega Corporation) was added thereto, followed by stirring for 20 minutes. Thereafter, the cells were measured using a luminescence measurement instrument (Envision; PerkinElmer Inc.). The values of positive control and negative control were regarded as 100% inhibition value and 0% inhibition value respectively to calculate the growth inhibition rate (%) of the respective compounds. As a result, the inhibition rates (%) of Sunitinib, Sorafenib and Vandetanib were 77%, 96% and 83% respectively.

The inhibitory effect of compounds against phosphorylation of KIF5B-RET in the FLAG-KIF5B-RETv1S/NIH3T3 cells was tested.
The FLAG-KIF5B-RETv1S/NIH3T3 cells were seeded at 2×10⁵ cells, and on the next day, Sunitinib, Sorafenib and Vandetanib were added thereto respectively at a concentration of 1 µM, and the resultant was cultured for 4 hours. Thereafter, the cells were lysed with a cell lysis solution (50 mM Tris·HCl (pH 7.4), 150 mM NaCl, 1% Triton X100, 5 mM EDTA, 5 mM EGTA, 1 mM NaVO₄, 1 mM DTT, a protease inhibitor cocktail complete). An SDS lysis solution was added thereto, immunoblotting was performed using anti-phosphorylated RET antibody (SANTA-CRUZ) specifically recognizing RET in which a 1062^{nd} tyrosine residue was phosphorylated and anti-RET antibody (Cell Signaling Technology, Inc.), and the phosphorylated amount and the total protein amount of about 110 Da of the phosphorylataed KIF5B-RET fusion polypeptide v1S were quantitatively calculated using the image analysis system (VersaDoc Imaging System; Bio-Rad Laboratories, Inc.). A value obtained by dividing the phosphorylated amount by the total protein amount was calculated, and an inhibition rate at the time when the compound was not added (when DMSO as a solvent of the compound was added) were taken as a control (0% inhibition rate) to calculate a phosphorylation inhibition rate at the time when the respective compound was added at 1 µM. As a result, the inhibition rates of Sunitinib, Sorafenib and Vandetanib were 57%, 94% and 74% respectively. From the results, it was understood that Sunitinib, Sorafenib and Vandetanib are KIF5B-RET fusion polypeptide v1S inhibitors.

The above results show that the KIF5B-RET fusion polypeptide inhibitor can inhibit the growth of cancer cells and tumors expressing the KIF5B-RET fusion polypeptide v1S.

It was demonstrated that if subjects of treatment using a KIF5B-RET fusion polypeptide inhibitor, for whom the treatment is expected to be effective, is screened using the detection method of the present invention, tailor-made treatment can be provided.

### [Industrial Applicability]

The detection method of the present invention is useful for detecting cancer patients positive for the fusion protein of the present specification. The detection kit and the primer set of the present invention can be used for the detection method.

### [Sequence Listing Free Text]

The numerical index <223> of the following sequence listing describes "Artificial Sequence". Specifically, the respective base sequences represented by SEQ ID NOs: 5 to 8, 11 and 12 of the sequence listing are primer sequences synthesized artificially.

## Claims

1. A detection method of a fusion gene of Kinesin family protein 5B (KIF5B) gene and rearranged during transformation (RET) gene or a fusion protein encoded by the fusion gene, comprising:
a step of detecting the presence of a polynucleotide encoding the following polypeptide or presence of the polypeptide in a sample obtained from a test subject:
a polypeptide that comprises an amino acid sequence having 90% or higher identities with the amino acid sequence represented by SEQ ID NO: 2 or 4 and has tumorigenicity.

2. The method according to claim 1,
wherein the polypeptide is a polypeptide that comprises the amino acid sequence represented by SEQ ID NO: 2 or 4 and has tumorigenicity, or a polypeptide that comprises an amino acid sequence formed by deletion, substitution and/or insertion of 1 to 10 amino acids in the amino acid sequence represented by SEQ ID NO: 2 or 4 and has tumorigenicity.

3. The method according to claim 1,
wherein the polypeptide is a polypeptide consisting of the amino acid sequence represented by SEQ ID NO: 2 or 4.

4. A kit for detecting a fusion gene of KIF5B gene and RET gene, comprising:
sense and antisense primers designed so as to be able to specifically amplify a polynucleotide encoding the following polypeptide:
a polypeptide that comprises an amino acid sequence having 90% or higher identities with the amino acid sequence represented by SEQ ID NO: 2 or 4 and has tumorigenicity.

5. The kit according to claim 4,
wherein the polypeptide is a polypeptide that comprises the amino acid sequence represented by SEQ ID NO: 2 or 4 and has tumorigenicity, or a polypeptide that comprises an amino acid sequence formed by deletion, substitution and/or insertion of 1 to 10 amino acids in the amino acid sequence represented by SEQ ID NO: 2 or 4 and has tumorigenicity.

6. The kit according to claim 4,
wherein the polypeptide is a polypeptide consisting of the amino acid sequence represented by SEQ ID NO: 2 or 4.

7. A primer set for detecting a fusion gene of KIF5B gene and RET gene, comprising:
a sense primer designed from a portion encoding the KIF5B gene; and
an antisense primer designed from a portion encoding the RET gene,
wherein the antisense primer consists of a nucleic acid molecule that is hybridized with a polynucleotide encoding the polypeptide according to any one of claims 1 to 3 under a stringent condition, and
the sense primer consists of a nucleic acid molecule that is hybridized with a complementary strand of the polynucleotide under a stringent condition.

8. A primer set of
a sense primer consisting of an oligonucleotide with at least any 16 consecutive bases located at base numbers 1 and 1725 of SEQ ID NO: 1 and
an antisense primer consisting of an oligonucleotide complementary to an oligonucleotide with at least any 16 consecutive bases located at base numbers 1726 and 2808 of SEQ ID NO: 1, or a primer set consisting of complementary strands thereof,
wherein a size of an amplification product amplified by the sense primer and the antisense primer is 1 kb or less.

9. A primer set of
a sense primer consisting of an oligonucleotide with at least any 16 consecutive bases located at base numbers 1 and 1725 of SEQ ID NO: 3 and
an antisense primer consisting of an oligonucleotide complementary to an oligonucleotide with at least any 16 consecutive bases located at base numbers 1726 and 2934 of SEQ ID NO: 3, or a primer set consisting of complementary strands thereof,
wherein a size of an amplification product amplified by the sense primer and the antisense primer is 1 kb or less.
